Europäisches Patentamt

European Patent Office

Office européen des brevets

(11) Publication number: 0 402 313 A1

# EUROPEAN PATENT APPLICATION

(21) Application number: 90810388.0

(51) Int. Cl.5: C07K 7/10, A61K 37/24

(22) Date of filing: 25.05.90

(30) Priority: 07.06.89 GB 8913089

(43) Date of publication of application:
12.12.90 Bulletin 90/50

(84) Designated Contracting States:
AT BE CH DE DK ES FR GB GR IT LI LU NL SE

(71) Applicant: Japat Ltd
Klybeckstrasse 141
CH-4057 Basel(CH)

(72) Inventor: Takai, Michihiro
Higashi-tokiwadai 2-17-6, Toyono-cho
Toyono-gun, Osaka(JP)
Inventor: Watakabe, Tadashi
Takatsukasa 3-2-18, Takarazuka-shi
Hyogo(JP)
Inventor: Okada, Toshikazu, Dr.
Gotenyama 4-22-17 Takarazuka-shi
Hyogo(JP)

(74) Representative: Schluep, Hans-Peter et al
c/o CIBA GEIGY AG Patentabteilung
Postfach
CH-4002 Basel(CH)

(54) Novel endothelin derivative.

(57) A novel endothelin-1 derivative represented by the following formula:

$$\text{Cys-Ser-Cys-Ser-Ser-Leu-}R_1\text{-Asp-Lys-Glu-Cys-Val-}$$
$$R_2\text{-Phe-Cys-His-Leu-Asp-Ile-Ile-Trp}$$

(I)

wherein $R_1$ represents norleucine, leucine, isoleucine or O-methyl homoserine and $R_2$ represents tyrosine 3-iodotyrosine, and wherein the iodine is cold or radioactive, and salts thereof; a pharmaceutical preparation comprising the derivative; and a biochemical agent comprising the derivative.

EP 0 402 313 A1

## NOVEL ENDOTHELIN DERIVATIVE

BACKGROUND OF THE INVENTION

1. Field of the Invention

The present invention relates to novel endothelin-1 derivatives, a process for the production thereof, and the use of the derivatives.

2. Description of the Related Art

Endothelin-1 is a potent vasoconstrictor peptide isolated and sequenced from the culture supernatant of porcine aortic endothelial cells (Yanagisawa M. et al. Nature 331, 411-415, 1988), which has the amino acid sequence: H-Cys$^1$-Ser$^2$-Cys$^3$-Ser$^4$-Ser$^5$-Leu$^6$-Met$^7$-Asp$^8$-Lys$^9$-Glu$^{10}$-Cys$^{11}$-Val$^{12}$-Tyr$^{13}$-Phe$^{14}$-Cys$^{15}$-His$^{16}$-Leu$^{17}$-Asp$^{18}$-Ile$^{19}$-Ile$^{20}$-Trp$^{21}$-OH. It is considered that the first Cys and the 15th Cys, as well as the third Cys and the 11th Cys, form disulfide bonds, respectively, resulting in the formation of two cystine residues in a molecule.

Endothelin-1 binds to a receptor of the vascular smooth muscle (Hirata Y. et al, Biochem. Biophys. Res. Commun. 125, 562-568, 1988) to contract the smooth muscle, resulting in a rise in blood pressure (Yanagisawa H. et al. Nature 331, 411-415, 1988). Namely, endothelin-1 is considered to be a trigger compound for vascular contraction, and to exhibit a cardiac stimulative action (Ishikawa T. et al. Am. J. Physiol., 255, H970-H973, 1988), and accordingly, it is expected that knowledge of the action mechanism of endothelin-1 will lead to an understanding of the mechanisms of hypertension and cardiac infarction. Moreover, it is expected that antagonists to a receptor of endothelin-1 are candidates as therapeutic agents for hypertension and cardiac infarction. Nevertheless native endothelin-1 is disadvantageous as both a biochemical agent and an ingredient of a pharmaceutical preparation in that it is unstable and thus has a short duration time in vivo.

This unstability is considered to be based on the susceptibility of the 7th methionine to oxidation. There have been attempts to improve the stability of a biologically active peptide by replacing the methionine residue with norleucine. C.B. Heward et al., (Biochem. Biophys. Res. Comm., 88 266 1979) disclose the replacing of methionine with norleucine in a β-melanocyto stimulating hormone, but here the iodination of the modified hormone was not successful.

SUMMARY OF THE INVENTION

Accordingly, the present invention relates to a novel endothelin-1 derivative wherein the 7th methionine in native endothelin-1 is replaced with another amino acid such as norleucine, and optionally the 13th tyrosine is iodinated, while maintaining desired biological properties.

More specifically, the present invention provides a endothelin-1 derivative represented by the following formula:

$$Cys-Ser-Cys-Ser-Ser-Leu-R_1-Asp-Lys-Glu-Cys-Val-$$

$$R_2-Phe-Cys-His-Leu-Asp-Ile-Ile-Trp$$

(I)

wherein $R_1$ represents norleucine, leucine, isoleucine or O-methyl homoserine and $R_2$ represents tyrosine or 3-iodotyrosine wherein the iodine is cold or radioactive.

The compounds of the invention can be in the free form but also in the form of their salts. As they contain free amino groups, the compounds of the invention can be in the form of acid addition salts. Suitable acid addition salts are in particular pharmaceutically acceptable salts with conventional pharmaceutically acceptable acids. Representative inorganic acids are hydrohalic acids (such as hydrochloric acid) and also sulfuric acid, phosphoric acid and pyrophosphoric acid. Representative organic acids are in particular arenesulfonic acids (such as benzenesulfonic or p-toluenesulfonic acid) or lower alkanesulfonic acids (such as methanesulfonic acid), as well as carboxylic acids such as acetic acid, lactic acid, palmitic acid, stearic acid, malic acid, tartaric acid, ascorbic acid and citric acid. As, however, the compounds according to the invention also contain free carboxyl groups they can also be in the form of salts with inorganic or organic bases, e.g. sodium, potassium, calcium or magnesium salts, or also ammonium salts derived from ammonia or a pharmaceutically acceptable organic nitrogen-containing base. However, as they contain at the same time free carboxyl groups and free amino groups, they can also be in the form of inner salts. Pharmaceutically acceptable salts are preferred.

The present invention also provides a pharmaceutical preparation comprising the above-mentioned endothelin-1 derivative wherein $R_1$ represents norleucine, leucine, isoleucine or O-methyl homoserine and $R_2$ represents tyrosine.

Moreover, the present invention provides a biochemical agent comprising the above-mentioned endothelin-1 derivative wherein $R_1$ represents norleucine, leucine, isoleucine or O-methyl homoserine and $R_2$ represents 3-iodotyrosine. The iodine may be cold iodine or radioactive iodine.

## PREFERRED EMBODIMENT OF THE INVENTION

The present endothelin-1 derivatives comprise two particular peptides, viz. a peptide wherein the 7th methionine of native endothelin-1 is replaced with norleucine, leucine, or isoleucine or O-methyl homoserine (derivative I), and a peptide wherein the 7th methionine of native endothelin-1 is replaced with norleucine, leucine, isoleucine or O-methyl homoserine and additionally the 13th tyrosine is replaced with 3-iodotyrosine (derivative II).

Both the derivatives I and II are remarkably stable in vivo and in vitro compared with native endothelin-1.

The iodination of the 13th tyrosine was compared between derivative I and native endothelin-1 (see Example 2 and Reference Example). In case of native endothelin-1,30 to 50 % of methionine was oxidized resulting in $Met(O)^7$-endothelin-1 and in a low yield of $^{13}I$-Tyr-endothelin-1, while derivative I gave no oxidized byproducts.

Further, both derivatives I and II exhibit a porcine aorta membrane binding activity and rat aorta contracting activity comparable to that of native endothelin-1.

The porcine aorta membrane binding activity was determined according to Biochem. Biophys. Res. Commun. 151, 1213, 1988, by the following procedure. Porcine aorta was dissected free from fatty tissue, minced with scissors, and homogenized with a homogenizer (Polytron®) in 10 volumes of buffer (10mM Tris-HCl, pH7.4, 0.25M sucrose). The homogenate was centrifuged at 1,000 x g für 15 min. The supernatant was filtered through 2 layers of nylon mesh, and followed by centrifugation at 48,000 x g for 30 min. The pellets were washed 3 times with the same buffer and kept frozen at -25° C until use within two months. Frozen membrane pellets were resuspended in 10 ml of binding assay buffer (Hank's balanced solution containing 0.1 % BSA (bovine serum albumin)) for each gram of original tissue wet weight. For receptor binding assays, 50 $\mu$l of $^{125}I$-endothelin-1 ([$Met^7$, $Tyr(^{125}I)^{13}$]-ET-1), final concentration 0.3 $\mu$M, was added to 900 $\mu$l of membrane suspension containing 50 $\mu$l of unlabeled ET-1 (0.3 $\mu$M final concentration for non-specific bindings) or endothelin-1 derivative to be tested, or 50 $\mu$l of buffer (for total binding). After incubation at 37° C for 60 min., the incubation mixtures were filtered through Whatman GF/B filters (presoaked in 1 % polyethyleneimine and set in a cell harvester, Brandel®) and washed with 3 ml of ice-cold binding assay buffer 3 times. The filters were counted for radioactivity by using an auto-gamma counter (Aloka ARC 361).

Activity of each peptide to inhibit the ability of $^{125}I$-endothelin-1 to bind to the porcine aorta membrane was as follows.

| Test peptide | $IC_{50}$ |
|---|---|
| Native endothelin-1 | 0.45 nM |
| 13-monoiodotyrosine endothelin-1 | 0.80 nM |
| Derivative I ($R_1$ = Nle) | 0.14 nM |
| Derivative II ($R_1$ = Nle) | 0.83 nM |

As seen from the above, the derivatives I and II exhibit an activity comparable to that of native endothelin-1.

The rat aorta contracting activity was determined according to Eur. J. Pharmacol. 53, 273 1977, by the following procedure.

Male sprague-Dawley rats (250-320 g body weight) were killed by decapitation. Rings of thoracic aorta (2 mm wide), cut close to the aortic arch, were suspended under resting tension of 2 g in a 50 ml organ bath containing physiological solution at 37° C and gassed with a mixture of 95 % $O_2$ and 5 % $CO_2$. Assays were performed 2 hours after dissection.

The activity of each peptide to contract isolated aorta of rat is set forth in the following table.

| Test peptide | $EC_{50}$ |
|---|---|
| Native endothelin-1 | 0.4 nM |
| 13-monoiodotyrosine endothelin-1 | 0.8 nM |
| Derivative I ($R_1$ = Nle) | 1.1 nM |
| Derivative II ($R_1$ = Nle) | 0.9 nM |

As seen from the above, the derivatives I and II exhibit an activity comparable to that of native endothelin-1.

Accordingly, the present derivative I is useful as an active ingredient of pharmaceutical preparations, such as hypertensors, cardiac stimulants, and the like.

Namely, the derivative I of the present invention can be used, for example, in the manufacture of pharmaceutical preparations that contain a therapeutically effective amount of the derivative together or in admixture with inorganic or organic, solid or liquid, pharmaceutically acceptable carriers that are suitable for parenteral, for example, intramuscular, intravenous, nasal, subcutaneous or intraperitoneal, administration.

For parenteral administration suitable injection or infusion solutions, preferably isotonic aqueous solutions or suspensions, are available it being possible to prepare these before use from, for example, lyophilized preparations that contain the active ingredient alone or together with a pharmaceutically acceptable carrier, for example mannitol. Such preparations may be sterilized and/or contain adjuncts, for example preservatives, stabilizers, wetting agents and/or emulsifiers, solubilizers, salts for regulating the osmotic pressure, and/or buffers.

The present pharmaceutical prepartions may contain, if desired, further pharmacologically valuable substances and are manufactured in a manner known per se, for example by conventional mixing, dissolving or lyophilizing processes. They contain approximately 0.1 % to 100 %, especially approximately 1 % to approximately 50 %, or, in the case of lyophilizates, up to 100 % of the active ingredient.

Depending upon the nature of the disease and the condition of the organism, parenteral doses of from approximately 1 μg to approximately 10 mg are given for the treatment of warm-blooded animals (humans and animals) weighing approximately 70 kg.

The invention concerns furthermore a method of treating hypotension in a mammal comprising administering to said mammal a therapeutically effective amount of a compound of the formula I in which $R_2$ represents tyrosine and of a pharmaceutically acceptable carrier.

The present derivative II is useful as a biochemical agent, especially for the study of the action mechanism of endothelin and analogues thereof. For this purpose, the iodine used as a label may be cold iodine or a radioactive iodine such as [125]I.

The iodine in the derivative II can be easily measured spectrometrically. Therefore, by comparing the plasma levels of derivative II with changes in blood pressure or electrocardiogram (ECG) after intravenous application of increasing doses of derivative II, endothelin-1 dependent sensitivity changes can be clarified. This procedure is applicable for diagnosis of diseases such as hypertension and cardiac ischemia.

The radioactively labelled derivative II is useful as a radioligand for radioimmunoassay to measure plasma levels of endothelin-1 in various patients suffering from hypertension, cardiac ischemia, cerebral ischemia, cerebral hemorrhage, bronchial asthma and renal failure. In addition the radioactively labelled derivative II is useful for the characterization of endothelin-1 receptors in the postmortem tissues from patients having suffered from the above diseases.

The present peptides can be prepared by a conventional procedure using commercial reagents and a commercial peptide synthesizer (for example ABI model 431). The chemical synthesis can be carried out in a manner known per se using corresponding amino acid units which are suitably protected. In particular, such a peptide synthesis comprises removing any protecting groups present in a compound corresponding to the compounds of the invention in which at least one of the amino, hydroxyl and/or carboxyl groups present carries a protecting group, and, if desired, converting an obtained peptide into a salt or converting an obtained salt into the free peptide. For example, the $\alpha$-amino group of all amino acids is protected by t-butoxycarbonyl (t-Boc), and functional groups of side chains are protected as follows: the thiol group of cysteine is protected by the 4-methylbenzyl group (4MeBzl), the $\epsilon$-amino group of lysine is protected by 2-chlorobenzyloxycarbonyl (ClZ), the $\beta$-carboxyl group of aspartic acid and the $\gamma$-carboxyl group of glutamic acid are protected by cyclohexyl ester (ocHex), the hydroxyl group of serine is protected by benzyl ether (Bzl), the phenolic hydroxyl group of tyrosine is protected by the 2-bromobenzyloxycarbonyl group (BrZ), the imidazole group of histidine is protected by the 4-toluenesulfonyl (Tos) group, and the indole group of tryptophan is protected by the formyl (CHO) group.

The synthesis of protected peptides is carried out by solid phase synthesis using an insoluble polystyrene resin derivative as a solid carrier. The Boc group is removed treating with trifluoroactic acid (TFA) before the condensation reaction at each step. Release from the carrier and deprotection of the synthesized peptide can be carried out simultaneously by an anhydrous hydrogen fluoride treatment.

The compounds of the invention are obtained in the free form or in the form of acid addition salts, inner salts or salts with bases. For example, the free compounds can be obtained in known manner from the acid addition salts or salts with bases. In turn, acid addition salts can be obtained from the free compounds by reaction with acids, e.g. with those acids which form the above-mentioned salts, and by evaporation or lyophilization. The inner salts can be obtained by adjusting the pH to a suitable neutral point.

## Examples

The present invention will now be illustrated by, but is by no means limited to, the following examples.

## Example 1. Synthesis of 7-Nle-endothelin-1

660 mg (0.5 mmol) of Boc-Trp(CHO)-OCH$_2$-phenyl-acetoamidemethyl (Pam) resin (a product of Applied Biosystems) was treated with 4.5 ml of 20 % trifluoroacetic acid (TFA) in CH$_2$Cl$_2$ for three minutes, and then treated with 10 ml of 50 % TFA in CH$_2$Cl$_2$ for 16 minutes. After the TFA treatment, the peptide resin was washed with methylene chloride, and neutralized by 10 % diisopropyl ethyl amine (DIEA) in N-methyl pyrrolidone (NMP). 481 mg (2 mmol) of Boc-Ile was dissolved in 3.4 ml of NMP, to this mixture were added 2 ml of 1 M 1-hydroxy benzotriazol (HOBt) and 2 ml of 1 M dicyclohexyl carbodiimide (DCCD). The mixture was stirred for 30 minutes, and then added to the amino acid (peptide) resin. After the reaction, the peptide resin was washed with NMP to be ready for the next step. One step was accomplished in about 1.8 hours.

The above step was repeated using 2 mmol each of the following protected amino acids:
Boc-Ile, Boc-Asp(OcHex), Boc-Leu, Boc-His(Tos), Boc-Cys(4MeBzl), Boc-Phe, Boc-Tyr(BrZ), Boc-Val, Boc-Cys(4MeBzl), Boc-Glu(OcHex), Boc-Lys(ClZ), Boc-Asp(OcHex), Boc-Nle, Boc-Leu, Boc-Ser(Bzl), Boc-Ser-(Bzl), Boc-Cys(4MeBzl), Boc-Ser(Bzl), Boc-Cys(4MeBzl), in this order, to obtain a protected peptide resin:
Boc-Cys(4MeBzl)-Ser(Bzl)-Cys(4MeBzl)-Ser(Bzl)-Ser(Bzl)-Leu-Nle-Asp(OcHex)-Lys(ClZ)-Glu(OcHex)-Cys-(4MeBzl)-Val-Tyr(BrZ)-Phe-Cys(4MeBzl)-His(Tos)-Leu-Asp(OcHex)-Ile-Ile-Trp(CHO)-OCH$_2$-Pam-resin.

4 ml of anisole and 36 ml of anhydrous hydrogen fluoride were added to the protected peptide resin, and the mixture was stirred at -2°C for 60 minutes. Subsequently, the mixture was concentrated under reduced pressure to obtain a residue, which was then precipitated by adding ethyl ether. After eliminating ethyl ether, 7.5 ml of 1,2-ethanedithiol and 2.5 ml of anhydrous hydrogen fluoride were added to the precipitate, and the mixture was stirred at -2°C for 30 minutes, and concentrated under a reduced pressure to obtain a residue. The residue was then precipitated by adding ethyl ether. The precipitate was put into 2 l of 0.1 M ammonium acetate/4 M urea (pH 8.0), and insoluble resin was filtered off. The filtrate was

5

oxidized in air at 10°C for 40 hours. The reaction mixture was desalted by application to Diaion HP-20 column (Mitsubishi Kasei Corp.) and elution with 80 % acetonitrile/1 N aqueous ammonia, the elute was concentrated under a reduced pressure and lyophilized, and the desired product was purified by high performance liquid chromatography (HPLC) to obtain 33 mg of a peptide represented by the following formula:

$$\text{Cys-Ser-Cys-Ser-Ser-Leu-Nle-Asp-Lys-Glu-Cys-Val-}$$

$$\text{Tyr-Phe-Cys-His-Leu-Asp-Ile-Ile-Trp.}$$

The thus-obtained peptide provided a single peak in HPLC on Toso TSK-Gel ODS 120T (4.6 mm x 150 mm) using 0.1 % aqueous TFA-CH$_3$CN (10 % - 60 % gradient, 30 min, 1 ml/min) as an eluent, and Asahipak ODP-50 (4.6 mm x 150 mm) using 50 mM phosphate buffer-CH$_3$CN (pH 7.8, 10% - 50 % gradient, 30 min, 1 ml/min) as an eluent, at duration times of 21.5 minutes and 16.5 minutes, respectively.

Result of amino acid analysis by acidolysis:

Asp 1.91(2), Ser 2.38(3), Glu ·1.00(1), Cys 1.78(4), Val 0.95(1), Ile 1.20(2), Leu 1.84(2), Tyr + Nle 1.73-(1 + 1), Phe 1.00(1), Lys 0.90(1), His 0.94(1), Trp not determined.

| FAB Mass Data | Theoretical: | $[M+H]^+ = 2473.07$ |
|---|---|---|
| | Found: | $[M+H]^+ = 2475.87$ |

In an analogous manner as described above 7-Leu-, 7-Ile- or 7-O-methyl homoserine-endothelin-1 are prepared if Boc-Leu, Boc-Ile and Boc-O-methyl-homoserine, respectively, is substituted for Boc-Nle in step 14 of the condensation reaction.

Example 2. Preparation of 7-Nle, 13 I-Tyr-endothelin-1

First, 240 μg of IoDo-Gen™ (tetrachloro-3α, 6β-diphenyl glycouril, a product of Pierce Ltd.) was dissolved in 1 ml of chloroform, and the solution was put into a glass tube and dried by a nitrogen flow. Then to this tube were added 5 mg of 7-Nle-endothelin in 4 ml of 50 mM phosphate buffer (pH 7.4) and 300 μg sodium iodide/200 μg of 50 mM phosphate buffer (pH 7.4), and the whole was stirred. The reaction was terminated by adding 3 ml of 0.9 % NaCl (pH 2.0), and the desired product was purified by HPLC and lyophilized.

This product provided a single peak at a duration time of 22.5 minutes in HPLC on Toso TSK-Gel ODS 120T (4.6 mm x 150 mm) using 0.1 % aqueous TFA-CH$_3$CN (10 % - 60 % gradient, 30 min, 1 ml/min) as an eluent. In mass spectrometry, this product provided a result that satisfactorily conformed to that of iodized 7-Nle-endothelin-1.

| FAB Mass Data | Theoretical: | $[M+H]^+ = 2601.93$ |
|---|---|---|
| | Found: | $[M+H]^+ = 2605.16$ |

Example 3. Infusion solution

10 micro grams of 7-Nle-endothelin-1 prepared in accordance with Example 1 are dissolved in one ml of physiological saline (10 % sodium chloride solution tradenamed KYORIN by Kyorin Pharm. Co.), and the solution is made isotonic and pH adjusted in a range of 6.0-7.0 by means of an aqueous dibasic potassium phosphate solution. The resulting solution is passed through a bacteriological filter and the filtered solution is filled into an ampoule under aseptic conditions.

The content of one ampoule is diluted with 250 ml of physiological saline as mentioned above to prepare a solution suitable for intravenous infusion.

Reference Example: Preparation of 13-I-Tyr-endothelin-1

Native endothelin-1 was synthesized by substantially the same procedure as described in Example 1, except that Boc-Met was used in place of Boc-Nle for the 7th amino acid to obtain, through the intermediate Boc-Cys(4MeBzl)-Ser(Bzl)-Cys(4MeBzl)-Ser(Bzl)-Ser(Bzl)-Leu-Met-Asp(OcHex)-Lys(ClZ)-Glu(OcHex)-Cys-(4MeBzl)-Val-Tyr(BrZ)-Phe-Cys(4MeBzl)-His(Tos)-Leu-Asp(OcHex)-Ile-Ile-Trp(CHO)-OCH$_2$-Pam, the following peptide:

Cys-Ser-Cys-Ser-Ser-Leu-Met-Asp-Lys-Glu-Cys-Val-

Tyr-Phe-Cys-His-Leu-Asp-Ile-Ile-Trp.

The above prepared peptide was then treated by the same procedure as described in Example 2 in order to obtain an endothelin-1 derivative labeled with iodine at the 13th tyrosine thereof.

After iodination reaction, 4 peaks were detected by HPLC. These peaks were identified as [Met(O)$^7$]-ET-1 (retention time (RT): 20.4 min), ET-1 (RT: 21.4 min), [monoiodo Tyr$^{13}$]-ET-1 (RT: 22.2 min) and [diiodo Tyr$^{13}$]-ET-1 (RT: 23.2 min).

| FAB Mass Data | Theoretical: | $[M + H]^+ = 2616.92$ |
|---|---|---|
| | Found: | $[M + H]^+ = 2620.32$ |

**Claims**

1. A peptide represented by the following formula:

Cys-Ser-Cys-Ser-Ser-Leu-R$_1$-Asp-Lys-Glu-Cys-Val-

R$_2$-Phe-Cys-His-Leu-Asp-Ile-Ile-Trp

(I)

wherein R$_1$ represents norleucine, leucine, isoleucine or O-methyl homoserine and R$_2$ represents tyrosine or 3-iodotyrosine, and wherein the iodine is cold or radioactive, and salts thereof.

2. A peptide of the formula I according to claim 1 in which R$_1$ represents norleucine and R$_2$ represents tyrosine or 3-iodotyrosine, and salts thereof.

3. 7-Nle-endothelin-1 according to claim 1.

4. 7-Nle-13-iodotyrosyl-endothelin-1 according to claim 1.

5. A pharmaceutical preparation comprising a peptide represented by the following formula:

$$
\begin{array}{c}
\overline{\text{Cys-Ser-Cys-Ser-Ser-Leu-}R_1\text{-Asp-Lys-Glu-Cys-Val-}} \\[2em]
R_2\text{-Phe-Cys-His-Leu-Asp-Ile-Ile-Trp}
\end{array}
$$

(I)

wherein $R_1$ represents norleucine, leucine, isoleucine or O-methyl homoserine, and $R_2$ represents tyrosine or a pharmaceutically acceptable salt thereof.

6. A biochemical agent comprising a peptide represented by the following formula:

$$
\begin{array}{c}
\overline{\text{Cys-Ser-Cys-Ser-Ser-Leu-}R_1\text{-Asp-Lys-Glu-Cys-Val-}} \\[2em]
R_2\text{-Phe-Cys-His-Leu-Asp-Ile-Ile-Trp}
\end{array}
$$

(I)

wherein $R_1$ represents norleucine, leucine, isoleucine or O-methyl homoserine, and $R_2$ represents 3-iodotyrosine, and wherein the iodine is cold or radioactive, or a pharmaceutically acceptable salt thereof.

7. A compound of the formula I according to claim 1 in which $R_2$ represents tyrosine for use in a method for the therapeutic treatment of the human or animal body.

Claims for the following Contracting States: ES, GR

1. A method for the preparation of a peptide represented by the following formula:

$$
\begin{array}{c}
\overline{\text{Cys-Ser-Cys-Ser-Ser-Leu-}R_1\text{-Asp-Lys-Glu-Cys-Val-}} \\[2em]
R_2\text{-Phe-Cys-His-Leu-Asp-Ile-Ile-Trp}
\end{array}
$$

(I)

wherein $R_1$ represents norleucine, leucine, isoleucine or O-methyl homoserine and $R_2$ represents tyrosine or 3-iodotyrosine, and wherein the iodine is cold or radioactive, and salts thereof, comprising removing any protecting groups present in a compound corresponding to the compounds of the invention in which at least one of the amino, hydroxyl and/or carboxyl groups present carries a protecting group, and, if desired, converting an obtained peptide into a salt or converting an obtained salt into the free peptide.

2. A method for the preparation of a peptide of the formula I according to claim 1 in which $R_1$ represents norleucine and $R_2$ represents tyrosine or 3-iodotyrosine, and salts thereof.

3. A method for the preparation of 7-Nle-endothelin-1 according to claim 1.

4. A method for the preparation of 7-Nle-13-iodotyrosyl-endothelin-1 according to claim 1.

European Patent Office

**EUROPEAN SEARCH REPORT**

Application Number

EP 90 81 0388

## DOCUMENTS CONSIDERED TO BE RELEVANT

| Category | Citation of document with indication, where appropriate, of relevant passages | Relevant to claim | CLASSIFICATION OF THE APPLICATION (Int. Cl. *f*) |
|---|---|---|---|
| A,D | NATURE, vol. 332, 31st March 1988, pages 411-415, London, GB; M. YANAGISAWA et al.: "A novel potent vasoconstrictor peptide produced by vascular endothelial cells" | | C 07 K    7/10<br>A 61 K   37/24 |
| A | TOXICON, vol. 26, no. 6, 1988, pages 543-548, Pergamon Press Plc, Oxford, GB; C. TAKASAKI et al.: "Sarafotoxins S6: several isotoxins from Atractaspis engaddensis (burrowing asp) venom that affect the heart" | | |
| A | PROC. NATL. ACAD. SCI. USA, vol. 85, September 1988, pages 6964-6967, Washington, DC, US; M. YANAGISAWA et al.: "Primary structure, synthesis, and biological activity of rat endothelin, an endothelium-derived vasoconstrictor peptide" | | |
| A | JOURNAL OF CARDIOVASCULAR PHARMACOLOGY, vol. 13 (suppl. 5), 6th March 1989, pages S197-S199, Raven Press, Ltd, New York, US; C.R. HILEY et al.: "Pressor effects of endothelin-1 and some analogs in the perfused superior mesenteric arterial bed of the rat" | | TECHNICAL FIELD SEARCHED (Int. Cl.*f*)<br><br>C 07 K<br>A 61 K |

The present search report has been drawn up for all claims

| Place of search | Date of completion of the search | Examiner |
|---|---|---|
| THE HAGUE | 03-09-1990 | DEFFNER C-A.E. |

EPO FORM 1503 03.82 (P0401)